# EUROPEAN PATENT APPLICATION

(11) **EP 3 190 411 A1**
(43) Date of publication of application: **12.07.2017**
(21) Application number: 16150311.5
(22) Date of filing: 06.01.2016
(51) Int. Cl.: G01N 33/49, A61B 5/15

(54) **ACCELERATED BLOOD COAGULATION PROCESS EMPLOYING A HIGH VACUUM**

(71) Applicant: Reapplix APS, 3460 Birkerød (DK)
(72) Inventor: Lundquist, Rasmus, 2700 Brønshøj (DK); Holm, Niels Erik, 3460 Birkerød (DK)
(74) Representative: Chas. Hude A/S

(57) **Abstract**

The present invention relates to a method comprising creating and maintaining a high vacuum environment inside the blood collection container has the effect of accelerating blood coagulation of a blood sample within said container, speeding up medical procedures dependent on fresh coagulated blood, and rapidly provides coagulated blood products effectively separated into blood serum and a blood clot absent of additives and contaminants which would make the blood unsuited for certain uses, such as blood banking and LeucoPatch®. The invention further relates to an apparatus and a kit suitable for accelerating blood coagulation in blood without requiring the use of additives.

Thereby, a pure and uncontaminated coagulated autologous blood product is achieved from a blood sample significantly faster than previously possible. When drawing blood without anticoagulant additive, such as when taking serum samples or blood for LeucoPatch® preparation, it is crucial to achieve fibrin coagulation as rapidly as possible, not only to save time, but to increase the quality of the blood clot and plasma separation and so purity and quality, especially when the blood sample is taken form a patient on anticoagulant therapy drugs like Xarelto®.

## Description

### [FIELD OF THE INVENTION]

The present invention relates to a method, apparatus and kit suitable for accelerating blood coagulation in blood without requiring the use of additives.

### [BACKGROUND]

After drawing blood from patients, coagulation of the fibrin in blood is desirable in some circumstances, such as for analysis of blood or for preparation of platelets and leucocyte rich fibrin patch. In these circumstances, it is desirable to complete fibrin polymerization as rapidly as possible to save processing time. Furthermore, the coagulation speed differ from patient to patient and certain patients have blood, which coagulates slowly, such as patients on anticoagulant therapy drugs like Xarelto®. For these patients with slow coagulation speed, the speed may be so slow as to impede autologous treatment and/or reduce the quality of blood clot and serum substantially, reducing treatment quality for potentially notably vulnerable patients, increasing the risk of e.g. needing perpetual treatment.

For these reasons there is a need for blood coagulation accelerators, and furthermore for blood collection equipment and methods, which provides an enhanced rate of blood coagulation without leaving any soluble or particulate material in the serum layer or in the clot on centrifugation, thus avoiding potential interference with clinical tests or contaminations when used autologous, such as for blood banking and for production of LeucoPatch®, as disclosed in EP2334307B1.

Known methods for accelerating and activating coagulation include exposure to diatomaceous earth and particles of inorganic silicates, and bio-chemicals such as ellagic acid and thromboplastin. A problem with particulate activators is that finely divided particles may not pellet completely with the clot and may thus contaminate the serum layer and interfere with certain blood uses. On the other hand, soluble biochemical activators can be disadvantageous because these cannot be easily separated from either the serum or blood clot and leaves the blood non-autologous. For specialized autologous applications, it is unacceptable to have either soluble activators or particulates in the cell mass of a blood clot.

In one line of commercial blood collection tubes, for example, a coating of silicate particles in polyvinylpyrrolidone (PVP, a water soluble polymer) is affixed to the inside of the tube. When blood enters the tube, the PVP dissolves and silicate particles are released to initiate clotting. This constitutes a contamination of said blood for autologous uses.

EP0740155A1 discloses serum tubes coated with material that activates the coagulation; however, said coating material may not adhere firmly to the serum tube surfaces and may therefore be carried forward into subsequent process steps. This constitutes a contamination of said blood sample, which is as problematic as the retention of chemical additives mentioned above.

EP0984279B1 discloses a method for accelerating blood coagulation relying on the bio-chemical additives thrombin and/or batroxobin. The resultant coagulated blood product may be useful for certain clinical tests, but the additives make it unsatisfactory for other uses such as autologous treatments.

### [SUMMARY OF THE INVENTION]

A first object of the invention is to solve the above-mentioned problems by providing accelerated blood coagulation, typically on whole blood and providing a blood clot and/or serum suitable for subsequent autologous and/or clinical use. This is achieved in the current invention essentially by creating and maintaining a high vacuum environment in at least the initial blood-drawing phase.

A method is employed, whereby a blood collection container (100) is evacuated of air, achieving a high vacuum in the interior volume (101) of the blood collection container (100).

The inventive method comprising creating and maintaining a high vacuum environment inside the blood collection container has the effect of accelerating blood coagulation of a blood sample within said container, speeding up medical procedures dependent on coagulation of blood absent of additives and contaminants which would make the blood unsuited for certain uses, such as blood banking and LeucoPatch®.

Thereby, a pure and uncontaminated coagulated autologous blood product is achieved from a blood sample significantly faster than previously possible. When drawing blood without anticoagulant additive, such as when taking serum samples or blood for LeucoPatch® preparation, it is crucial to achieve fibrin coagulation as rapidly as possible, especially when the blood sample is taken form a patient on anticoagulant therapy drugs like Xarelto®.

Any container capable of holding a vacuum is suitable for use in the current invention. Most conveniently, a blood collection container that is suitable to generate and maintain the high vacuum of the current invention is suitable.

In one embodiment of the invention, this is provided by a blood collection kit of parts (4000) comprising a blood collection container (100). Said blood collection container (100) comprises a bottom wall (102) and a side wall (103) defining an open end, a stopper (104) in said open end, said stopper (104) further comprising a puncturable self-sealing septum (105) or valve, said elements defining an interior volume (101) of said container (100). The blood collection container may be any container having a closed end and an open end; for example bottles, vials, flasks and the like. Any material or aggregate of materials capable of maintaining a high vacuum with at least a transitory durability and suitable for subsequent clinical use may be used in the blood collection container (100), including but not limited to glass, polyethylene terephthalate (PET) and/or polystyrene (PS).

In one embodiment of the invention, said blood collection container is at least transitorily vacuum durable.

Having a vacuum durable blood collection container allows the vacuum to better persist over time, which allows easier handling and more efficient production, such as allowing an industrially produced blood collection container to retain a vacuum during subsequent processing and transport.

In one embodiment of the invention, a part of the inner surface of said bottom wall (106) and/or a part of the inner surface of said side wall (107) of the blood collection container (100) has been plasma treated. In another embodiment, the entire inner surface of the walls (106, 107) has been plasma treated. In either embodiment, the inside of said stopper (108) may optionally be also plasma treated. Likewise, parts placed inside the container may also be plasma treated.

The plasma may be generated from any suitable process gas. A representative but not limiting list of suitable process gases includes nitrogen, ammonia, carbon dioxide, sulfur dioxide, air and oxygen wherein air and oxygen are preferred. The tube may be placed open end up between the electrodes of a conventional plasma generator equipped with a pressure gauge, a gas inbleed and a vacuum connection. Suitable electrodes may be of any conducting material, although stainless steel and aluminum are preferred. The width and shape of the electrodes is not critical. Any suitable ionizing plasma may be used, as, for example, a plasma generated by a corona discharge or a glow discharge.
Likewise, atmospheric-pressure plasma treatment or other technologies used for surface treatment include in-line atmospheric (air) plasma, flame plasma, and chemical plasma systems may be used.

Alternatively or additionally, the insides may be abraded in any convenient way. The surface may be roughened by any conventional chemical or mechanical method, or during the tube forming process. Most conveniently, the surface is merely rubbed with an abrasive, such as with sand or emery paper. No limitation is placed on the grit of the abrasive.

Plasma treating and/or abrading increases surface area and friction with the blood, and are known in the art to activate coagulation. Combining the high vacuum with another coagulation activator useful for autologous use further increases coagulation speed, increasing blood product quality and time for post-treatment care.

In an embodiment of the current invention, the blood collection container (100), having in its interior volume (101) a high vacuum, is inserted into a first container means (200) characterized in that it possesses at least transitory vacuum durability and furthermore, that it can be subjected to a sterilization procedure and retain at least transitory vacuum durability. The first container means (200) may take any shape, preferably being a bag or sack and consist of any type of material or aggregate of types of material, a representative but not limiting list of materials would include plastics, cardboard, glass, aluminum and/or aluminum foil. Said first container means (200) comprises at least one material affording it transitory vacuum durability, and furthermore allows sterilization of said first container means (200) and its interior volume without degrading its vacuum durability to less than a transitory vacuum durability, preferred actual materials being dependent on sterilization method. In a preferred embodiment of the invention, the first container means (200) comprises at least polyethylene terephthalate (PET) or polystyrene (PS).

Insertion into a first container means improves the durability of the high vacuum inside the blood collection container, since the pressure drop from the outside air to the blood collection container now has to permeate two barriers. This improves industrial applicability in a cost-efficient way, since the blood collection container does not have to be produced with specifications to maintain its high vacuum during handling or transport, but only during use.

In one embodiment of the invention, the first container means (200) containing the blood collection container (100) has been sterilized. Any method of sterilization may be used, but typically the most convenient techniques includes heat (or heat and pressure) sterilization, chemical sterilization, radiation sterilization, or any combination thereof. In a preferred embodiment, gamma-ray sterilization is utilized.

Sterilization either before or after insertion into a first vacuum container means is performed to significantly reduce risk of contamination during clinical use of the blood collection container and so fulfill regulatory and safety requirements.

Because the high vacuum imposes a higher pressure differential on the blood collection container of this invention when compared to blood collection containers of the art, contaminations are more likely to be introduced simply by power of diffusion through imperfections in its construction, materials and/or seams. By inserting the blood collection container into a first vacuumed container means and sterilizing it inside this said first vacuumed container means, the likelihood of any partial depressurization and its associated risk of contamination of the inside of the blood collection container is eliminated or reduced.

In one embodiment of the invention, sterilization of the first container means (200) containing the blood collection container (100) is followed by inserting the first container means (200) containing the blood collection container (100) into a second container means (300), characterized in that said second container substantially durably maintains a vacuum, said container means (200) is then emptied of air and sealed. Preferably, said second container means (300) comprises at least one material enabling said second container means (300) to substantially durably maintain a vacuum. Said second container means (300) may consist of any type of material or aggregate of types of material, a representative but not limiting list of materials would include plastics, cardboard, glass and/or aluminum. In a preferred embodiment of the invention, the second container means (300) comprises at least aluminum or aluminum foil.

Sterilizing the blood collection container (100) inside the first container means (200) with limited vacuum durability that is unfit for long time storage provides a transitory vacuum durability even through sterilization. To increase vacuum durability, the first container means (200) containing the blood collection container (100) is inserted into a second container means (300) chosen for substantial vacuum durability and handling durability.

This dual-sealing allows a blood collection container to be produced which meets regulatory and safety requirements while attaining and maintaining a substantially durable vacuum through transportation and storage in a way that meets economic and industrial specifications.

In an embodiment of the invention, the blood collection container (100) is sterilized before insertion into the first container means (200).

With a sufficiently high quality blood collection container, said high quality pertaining to it being substantially vacuum durable after a sterilization procedure, it may not be necessary to dual-seal it to achieve the desired sterility and vacuum durability. Sealing it once and doing so after sterilization is then sufficient for handling and vacuum durability purposes.

The blood collection container may be prepared with a high vacuum at any convenient time prior to, or during coagulation. An inexhaustible list of times are during manufacture; at a time prior to drawing blood recognizing the vacuum durability of the used blood collection container ensuring that at least a high vacuum remains when drawing blood; and at a time prior to blood coagulation recognizing the vacuum durability of the used blood collection container ensuring that at least a high vacuum remains when coagulating blood. In an embodiment of the invention, it is important to expose the blood to the high vacuum during blood draw to maximally agitate the blood and so initiate and/or accelerate coagulation.

For economic reasons as well, applying the high vacuum prior to drawing blood is preferable, since the vacuum serves to draw the blood from the patient more quickly, and a vacuum would otherwise be applied to the blood collection container twice, or alternatively, the blood draw would be unnecessarily slow.

The high vacuum of the blood collection container may be prepared in any convenient way, such as by using a vacuum pump. The vacuums of the first and second container means may likewise be applied in any convenient way.

In one embodiment of the invention, the blood coagulation inside the first blood collection container (100) is further accelerated by at least one coagulation-accelerating object inside of it, which through this exposure accelerates blood coagulation. For example, it may expose the blood to one or more glass surfaces, glass having long been known in the art to promote coagulation. Such objects may be one or more glass beads.

By exposing the blood to a further coagulating agent, the coagulation time can be further decreased, ensuring the shortest possible process time and so increasing blood product quality and time for post-treatment care.

In one embodiment of the invention, the first blood collection container (100) comprises a blood sample (406) and a high vacuum.

By combining a blood sample with a high vacuum inside a given environment, conveniently a blood collection container, an increased coagulation speed is ensured, attaining a shorter process time and so increasing blood product quality and time for post-treatment care.

The blood may be drawn into the blood collection container by any means used in the art. In one embodiment of the invention, the blood collection kit of parts (4000) further comprises a double-ended needle (400) for drawing blood from a patient into the first blood collection container (100), comprising a first needle-end (401), a second needle-end (402) and an intermediate tube (403), said double-ended needle (400) comprising an interior volume (404) running the axial length of the double-ended needle (400).

In one embodiment of the invention, a first needle-end (401) of said double-ended needle first enters a convenient blood-filled cavity, typically of a patient and preferably a vein of said patient. After the first needle-end (401) has entered its designated blood-filled cavity, a second needle-end (402) punctures the puncturable septum (105) of the blood collection container (100). The vacuum inside the interior volume (101) of the blood collection container (100) then draws the blood from the patient by way of a pressure-differential.

It is important that drawing of the blood is interrupted before the interior volume (101) is depressurized to less than a high vacuum.

In another embodiment of the invention, the double-ended needle (400) may instead be a catheter inserted into the circulatory system of a patient.

In one embodiment of the invention, the inner volume (404) defined by the double-ended needle's (400) inner surface is such that bringing it into fluid communication with the interior volume of the blood collection container (101) retains the high vacuum in the interior of the blood collection container. This consequently builds a high vacuum in the interior volume of the double-ended needle (404).

Achieving this combined high vacuum requires a comparatively higher initial vacuum in the blood collection container (101) as well as a controlled volume of the double-ended needle (404), both taken together ensuring a controlled depressurization before exposure to the blood. However, the balance of these two may take any form. For example, creating an extremely high vacuum in a large blood collection container allows a comparatively larger double-ended needle, and conversely, a double-ended needle with a small volume will allow for a lower grade vacuum and/or a smaller blood collection container.

In one embodiment of the invention, the double-ended needle has a fixed low interior volume Vᵣ or Vₐ so as to limit the depressurization of the blood collection container during use. For example, with a double-ended needle volume of 10 % of the blood collection container of 100 ml and a prepared initial vacuum of 99 %, the combined volume being 110 ml would hold 11ml of air, or 90 % vacuum. A low inner volume (404) of the double-ended needle thus ensures that the vacuum exerts a comparatively greater effect on the blood sample (405), speeding up the coagulation and so increasing time for post-treatment care.

In one embodiment of the invention, the double-ended needle further comprises coagulation accelerating objects in its interior volume (404). By exposing the blood to agitating objects here, process time is further decreased while the depressurization as consequence of bringing the interior volumes of the blood collection container and the double-ended needle into contact is lessened.

### [BRIEF DESCRIPTION OF DRAWINGS]

Fig. 1 is a side view of the evacuated blood collection container means according to the present invention;
Fig. 2 is a view of the evacuated blood collection container means inserted into a first vacuum-sealed container means according to the present invention;
Fig. 3 is a view of the evacuated blood collection container means inserted into a first vacuum sealed container means inserted into a second vacuum sealed container means according to the present invention;
Fig. 4 is a side view of the blood collection container means while drawing blood through a double-ended needle.
Fig. 5 details measurement data demonstrating the effects of degrees of vacuum on coagulation of blood.

### [DETAILED DESCRIPTION OF DRAWINGS]

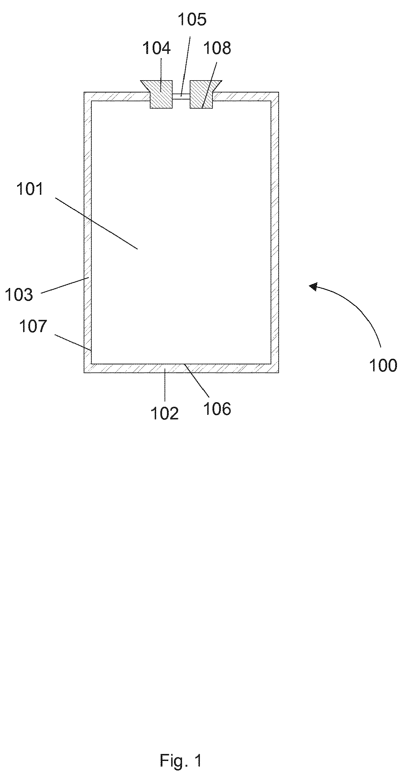

Fig. 1 describes a side view of a blood collection container (100) comprising a bottom wall (102), a side wall (103) forming a container with an open end and a closed end. A stopper (104) is fitted in said open end, said bottom wall (102), side wall (103) and stopper (104) enclosing an interior volume (101) of said blood collection container (100), said stopper comprising at least a mechanism for securely sealing said open end and maintaining a pressure differential, in a preferred embodiment making the blood collection container at least transitorily vacuum durable. Said stopper (104) further comprises a self-sealing puncturable septum (105) or a valve allowing the emptying of air from the interior volume (105) through any convenient method, such as a vacuum pump, said self-sealing puncturable septum (105) or valve further allowing the insertion of a needle or other tube-end without simultaneously allowing atmospheric air into the interior volume (101). This allows passage of blood through a needle into the blood collection container while maintaining a high vacuum in the interior volume (101).

Further depicted in fig. 1 is an inner surface of said bottom wall (106), an inner surface of said side wall (107) and an inner surface of said stopper (108), said inner surfaces all facing and in contact with said interior volume (101). These or some of these surfaces may be plasma treated and/or abraded to further accelerate coagulation speed.

The blood collection container is prepared for use by inserting the stopper into its designated position in the open end of the blood collection container (100), and emptying the blood collection container of air through the self-sealing puncturable septum (105) or valve, said emptying of air further securing said stopper in its position by exerting a downward pressure due to the pressure differential over said stopper.

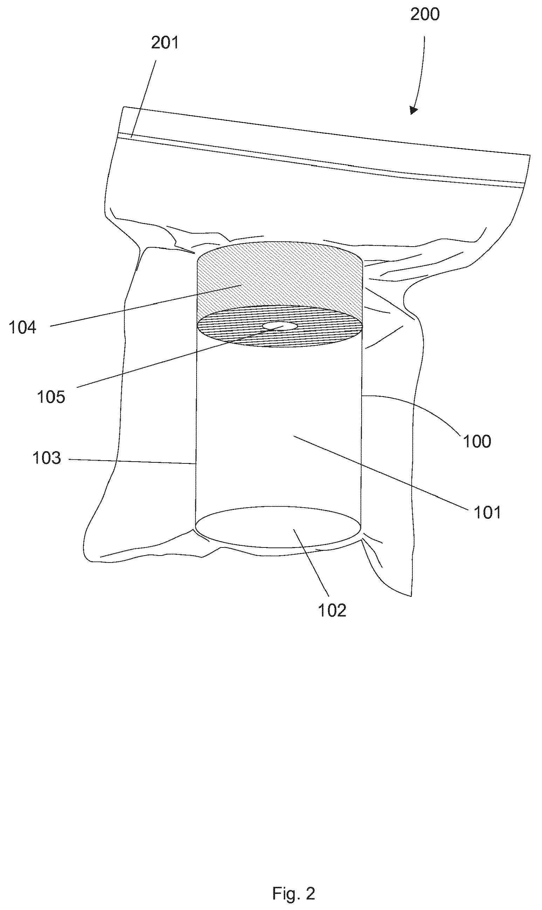

Fig. 2 is a side view of a blood collection container (100) inside a vacuum-sealed first container means (200), said first container means being at least transitorily vacuum durable, said blood collection container comprising a bottom wall (102), a side wall (103) defining a container with an open and a closed end, and a stopper (104) in said open end, said stopper further comprising a puncturable septum (105) or a valve for emptying the enclosed interior volume (101) of air, creating a high vacuum, said puncturable septum or valve further allowing drawing blood into the blood collection container without simultaneously allowing atmospheric air into the interior volume (101).

The blood collection container is inserted into the first container means (200), which is emptied of air ensuring that it tightly encloses said blood collection container whereupon said first container means is vacuum sealed, lowering the pressure differential over the bottom wall, side wall and stopper of said blood collection container, improving the effective vacuum durability of said blood collection container. Any convenient sealing method may be used and in one embodiment, when a convenient vacuum has been achieved inside the first container means, it is heat sealed at the sealing line (201).

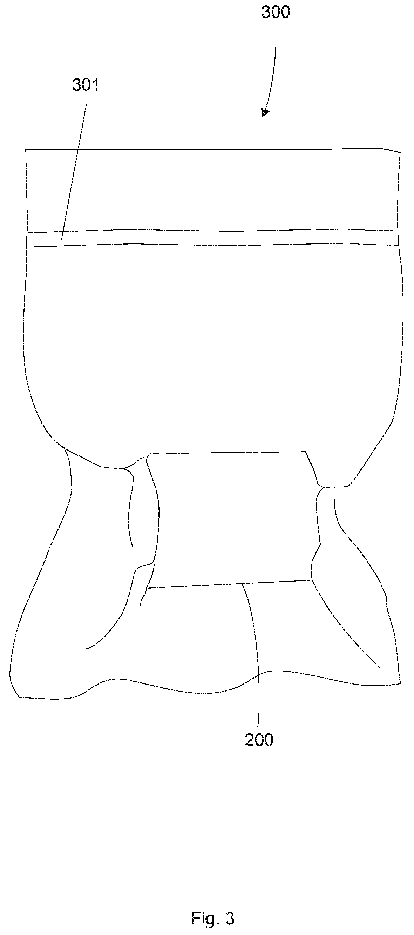

Fig. 3 is a side view of a vacuum sealed second container means (300) being substantially vacuum durable, said second container means comprising a first container means (200) further comprising a blood collection container prepared with a high vacuum.

The three containers taken together, the blood collection container, the first container means (200) and the second container means (300), make up a blood collection kit of parts prepared for transportation, storage and handling.

A first container means (200) vacuum sealed and comprising a blood collection container is preferably sterilized, after which it is inserted into a second container means (300), said second container means is then emptied of air ensuring that it tightly encloses said first container means, whereupon said second container means is vacuum sealed, lowering the pressure differential over the enclosed vacuum barriers, improving the effective vacuum durability of said blood collection container. In one embodiment, when a convenient vacuum has been achieved inside the second container means such as a high vacuum, it is heat sealed at the sealing line (301).

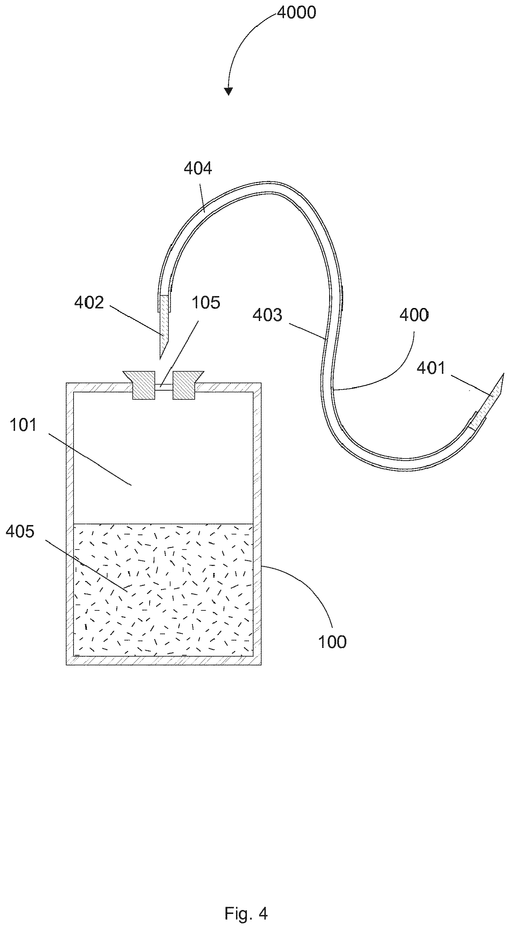

Fig. 4 is a side view of a blood collection kit of parts (4000), the kit of parts comprising a blood collection container (100) and a double-ended needle (400) after drawing blood from a patient and disassembling the kit of parts for further processing of the blood.

The blood collection container (100) comprises a bottom wall (102), a side wall (103) defining a container with an open and a closed end, and a stopper in said open end, said stopper further comprising a puncturable septum or a valve for emptying the enclosed interior volume (101) of air, creating a high vacuum, said puncturable septum or valve further allowing drawing blood into the blood collection container without simultaneously allowing atmospheric air into the interior volume (101), the. The blood collection container further comprises a blood sample (405).

The double-ended needle comprises a first needle-end (401), a second needle-end (402), an intermediate tube (403) and an inner volume (404) running the axial length of said double-ended needle, the inner volume allowing the transport of blood from the patient to the blood collection container. In one embodiment of the invention, the inner volume further comprises coagulation accelerating objects, such as one or more glass beads.

In one embodiment of the invention, the blood collection kit of parts further comprises a first container means (200) and a second container means (300).

A high vacuum may be introduced into the blood collection container by any convenient method, preferably a blood collection kit of parts (4000) is unpackaged, allowing access to the blood collection container (100) from within a first and a second container means, the blood collection container being previously prepared with a high vacuum. The first needle-head (401) then enters a convenient volume of the patient, typically a vein, after which blood begins running through the double-ended needle's interior volume (404) and simultaneously blocks fluid communication through said first needle-end (401). A second needle-end (402) then punctures the self-sealing septum of a blood collection container (100), which brings the high vacuum interior volume of the blood collection container (101) into fluid communication with the inner volume of the double-ended needle (404), this fluid communication decreasing the pressure in the inner volume of the double-ended needle (404), and so actuates an accelerated blood draw.

Preferably, the second needle-end is withdrawn from the self-sealing puncturable septum at a time when the interior volume of said blood collection container retains a high vacuum or at least remains lower than 1013,25 mBar / ambient pressure. The situation illustrated in fig. 4 is this lastly described where blood has been drawn and the double-ended needle has been removed from the blood collection container and from the patient.

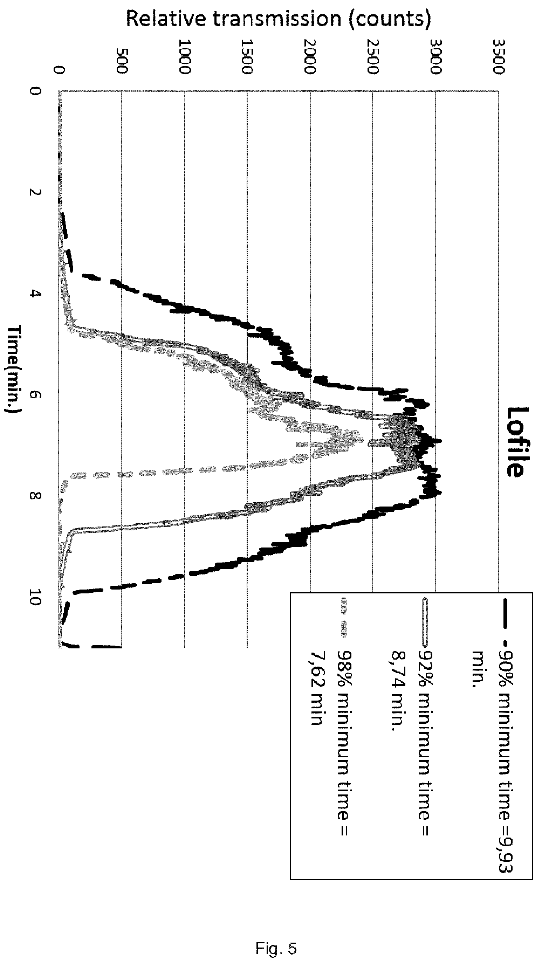

Fig. 5 is measurement data of the different effects on coagulation speed by using 90% (101 mBar), 92% (81 mBar) and 98% (20 mBar) vacuum in a blood collection container during coagulation while inserted into an active centrifuge, where the coagulation process is observed by way of the translucency of the blood sample.

In a first phase, translucency continually increases as the blood separates and blood plasma remains in the uppermost part due to it comprising elements with relative to the rest of the blood lower Svedberg values, which means forces like gravity and the centripetal force acts less strongly on these elements (and may for the sake of this invention be thought of as an alternative property to mass).

In a second phase, fibrin in the blood plasma begins polymerizing, reducing translucency as the blood plasma becomes more opaque. This reduces transmission counts. Table 1 describes the events shown in fig. 5:

**Table 1.**

| | 90 % | 92 % | 98 % |
|---|---|---|---|
| peak transmission count at | 7,94 minutes | 7,45 minutes | 6,91 minutes |
| post-peak lowest transmission count at | 9,93 minutes | 8,74 minutes | 7,62 minutes |

### [DEFINITIONS]

High Vacuum = A pressure of no more than 255 mBar, 253,31 mBar, 202,65 mBar, 151,99 mBar, 101,33 mBar, 91,19 mBar, 81,06 mBar, 70,93 mBar, 60,80 mBar, 50,66 mBar, 40,53 mBar, 30,40 mBar, 20,27 mBar, 10,13 mBar, 5,07 mBar, 1,01 mBar, 0,10 mBar or less, and where the preferable range of pressure is 0,10 mBar - 101,33 mBar or less.

In another embodiment of the invention, said high vacuum corresponds to a vacuum of at least 75% (253,31 mBar), 80% (202,65 mBar), 85% (151,99 mBar), 90% (101,33 mBar), 91% (91,19 mBar), 92% (81,06 mBar), 93% (70,93 mBar), 94% (60,80 mBar), 95% (50,66 mBar), 96% (40,53 mBar), 97% (30,40 mBar), 98% (20,27 mBar), 99% (10,13 mBar), 99,5% (5,07 mBar), 99,9% (1,01 mBar) or even 99,99% (0,10 mBar) or above, as measured linearly where 0% vacuum is 1013,25 mBar and 100% vacuum is 0 mBar, and where the preferable range of vacuum is 90% - 99,99 % or above.

Vᵣ, being the inner volume (404) of a double-ended needle (400) = 1%, 2%, 3%, 4%, 5%, 6%, 7%, 8%, 9%, 10% or less relative to the volume of the blood collection container (100).
Vₐ = 0,1 ml, 0,2 ml, 0,3 ml, 0,4 ml, 0,5 ml, 0,6 ml, 0,7 ml, 0,8 ml, 9 ml or 1,0 ml.

Vacuum Durability = a quality of a boundary enclosing an environment defining its ability to maintain a percentage of a pressure differential over said boundary over time, specifically where there is a pressure drop from the exterior to the enclosed environment. Two distinct degrees of vacuum durability is described; transitory vacuum durability and substantial vacuum durability, where:
A boundary with a transitory vacuum durability maintains at least 10% of its pressure differential over 24 hours, 48 hours, 72 hours, 96 hours, 120 hours, 144 hours, 168 hours or more, where the initial pressure inside the enclosed environment is a high vacuum and relative to 1013,25 mBar at ambient temperature outside.

A boundary with a substantial vacuum durability maintains at least 98 % of its pressure differential over 1 week, 2 weeks, 3 weeks, 4 weeks, 6 weeks, 8 weeks, 12 weeks or more, where the initial pressure inside the enclosed environment is a high vacuum and relative to 1013,25 mBar at ambient temperature outside.

Blood collection container = a container specifically designed for storage and collection of blood immediately after drawing blood from a patient. Such blood collection containers may be used in subsequent treatment of the blood, such as in producing LeucoPatch®, or it may only be used for transportation and storage of the blood for later use. Blood collection containers are most conveniently produced in glass, polyethylene terephthalate (PET) and/or polystyrene (PS).

## Claims

1. A method for accelerated blood coagulation, comprising: step a) removing air inside a blood collection container (100), attaining an internal pressure of 255 mBar or less inside said blood collection container (100).

2. A method according to claim 1, wherein a pressure of 205 mBar or less is achieved inside said blood collection container (100).

3. A method according to claim 1, wherein a pressure of 155 mBar or less is achieved inside said blood collection container (100).

4. A method according to claim 1, wherein a pressure of 105 mBar or less is achieved inside said blood collection container (100).

5. A method according to any of claims 1 - 4, where step a) is subsequently followed by: b) inserting the said blood collection container (100) into a first container means (200) and vacuum sealing said first container means (200) containing said blood collection container (100).

6. A method according to claim 5, where step b) is subsequently followed by: c) sterilizing said first container means (200) and said blood collection container (100) inside said first container means (200).

7. A method according to claim 6, where step c) is followed by: d) inserting said first container means (200) containing said blood collection container into a second container means (300) and vacuum sealing said second container means containing said first container means (200) containing said blood collection container (100).

8. A method according to claim 6-7, where step c) precedes step b).

9. A blood collection kit of parts (4000) comprising at least a blood collection container (100), said blood collection container having a bottom wall (102) and a side wall (103) defining an open end, a stopper (104) in said open end, said stopper (104) further comprising a puncturable self-sealing septum (105) or valve, said elements defining an interior volume (101) of said blood collection container (100), **characterized in that** the interior volume (101) has been prepared with a pressure in its inner volume (101) of no more than 255 mBar.

10. A blood collection kit of parts (4000) according to claim 9, wherein said container (100) is capable of retaining at least 10% of the pressure differential between the high vacuum in its interior volume (101) relative to ambient temperature and pressure on the outside after 24 hours.

11. A blood collection kit of parts (4000) according to any of claims 9 - 10, where at least part of the inner surfaces (106, 107, 108) of said blood collection container (100) surrounding the interior volume has been plasma treated and/or abraded.

12. A blood collection kit of parts (4000) according to any of claims 9 - 11, further comprising a blood coagulation acceleration agent, such as one or more glass beads, inside the blood collection container.

13. A blood collection kit of parts (4000) according to any of claims 9 - 12, wherein said kit of parts further comprises a first container means (200) and a second container means (300), said second container means is capable of retaining at least 98% of the pressure differential between the vacuum in its interior volume relative to ambient temperature and pressure on the outside after 1 week and vacuum sealed around said first container means (200), which is capable of retaining at least 10% of the pressure differential over its enclosing walls after 24 hours and is vacuum sealed around said blood collection container (100), which is prepared with a pressure of no more than 255 mBar.

14. A blood collection kit of parts (4000) according to claim 13, where the first container means (200) comprises of PU and/or PET and the second container means (300) comprises of aluminium or aluminium foil.

15. A blood collection kit of parts (4000) according to any of claims 9-14, further comprising a double-ended needle (400), said double-ended needle comprising a first needle-end (401), a second needle-end (402), an intermediate tube (403), and an inner volume (404) running the axial length of said double-ended needle, where the combination of the degree of vacuum prepared in said interior volume of the blood collection container (101) and said inner volume of said double-ended needle (404) is such that bringing the two inner volumes (101, 404) into fluid communication retains a pressure of 255 mBar or less inside the inner volumes (101, 404).

16. A blood collection kit of parts (4000) according to claim 15, wherein said double-ended needle (400) further comprises a blood coagulation acceleration agent, such as one or more glass beads, inside its interior volume (404).
